(19) Europäisches Patentamt
European Patent Office
Office européen des brevets

(11) Veröffentlichungsnummer: **0 170 896**
**B1**

# (12) EUROPÄISCHE PATENTSCHRIFT

(45) Veröffentlichungstag der Patentschrift: 24.05.89

(51) Int. Cl.⁴: **C 07 F 9/38**, A 61 K 31/66

(21) Anmeldenummer: 85108380.8

(22) Anmeldetag: 05.07.85

(54) Neue 1-Hydroxy-1,1-diphosphonsäureverbindungen, Verf. zu ihrer Herstellung u. pharmakologische Zubereitungen, insb. zur Behandlung von Knochentumoren.

(30) Priorität: 13.07.84 DE 3425812

(43) Veröffentlichungstag der Anmeldung: 12.02.86 Patentblatt 86/7

(45) Bekanntmachung des Hinweises auf die Patenterteilung: 24.05.89 Patentblatt 89/21

(84) Benannte Vertragsstaaten: AT BE CH DE FR GB IT LI NL SE

(73) Patentinhaber: **Henkel Kommanditgesellschaft auf Aktien, Postfach 1100 Henkelstrasse 67, D-4000 Düsseldorf-Holthausen (DE)**
Patentinhaber: **Deutsches Krebsforschungszentrum Stiftung des öffentlichen Rechts, Im Neuenheimer Feld 280, D-6900 Heidelberg 1 (DE)**

(72) Erfinder: Blum, Helmut, Bertha-von-Suttner-Strasse 30, D-4000 Düsseldorf 13 (DE)
Erfinder: Greb, Wolfgang, Dr. Dr., Am Fronberg 11, D-4000 Düsseldorf-Kaiserswerth (DE)
Erfinder: Möller, Hinrich, Dr., Schumannstrasse 11, D-4019 Monheim (DE)
Erfinder: Schmähl, Dietrich, Prof. Dr. med., Sandwingert 57, D-6900 Heidelberg (DE)
Erfinder: Schnegelberger, Harald, Dr., Stieglitzweg 2, D-5653 Leichlingen (DE)
Erfinder: Sinn, Hansjörg, Dr., Ahornweg 10, D-6908 Wiesloch 4 (DE)
Erfinder: Wingen, Franz, Schulzengasse 6, D-6900 Heidelberg (DE)

(56) Entgegenhaltungen:
DE-A- 2 405 254

**JOURNAL OF MEDICINAL CHEMISTRY, Band 22, Nr. 10, Oktober 1979, Seiten 1267-1269, American Chemical Society, US; A. PANTHANANICKAL et al.: "Structure-activity relationship of aniline mustards acting against B-16 melanoma in mice"**
**H. Hamacher et al., Arch. Pharm., 311, 184 (1978), Seiten 185-195**
**S. Sieber et al., Cancer Treatment Reports, Vol. 60, No. 3, (1976), Seiten 217-219**
**Benedict et al., J. Canc. Res. 37, S. 2209-2213 (1977)**
**Singh et al., J. Canc. Res. 43, S. 577-584 (1983)**
**Advances in Cancer Chempotherapy, H. Umezawa et al., (EDS.), Japan, Sci. Soc. Press, Tokyo/Univ. Park Press, Baltimore, Seiten 283-296 (1978)**

(56) Entgegenhaltungen: (Fortsetzung)
**B. L. POOL et al.: "IN VIVO AND IN VITRO INVESTIGATIONS ON BILOGICAL EFFECTS OF AROMATIC BIS-(2-CHLOROETHYL)AMINO-BISPHOSPHONIC ACIDS; NEW AGENTS PROPOSED FOR OSTEOSARCOMA; TOXICITY AND GENOTOXICITY IN LIVER AND BONE MARROW; MUTAGENICITY IN S. TYPHIMURIUM" zur Veröffentlichung in Toxicology letters und dort im Druck**

**EP 0 170 896 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Cytostatika zur Behandlung von Knochentumoren sind – soweit bisher bekannt ist – tumorunspezifisch und erfordern daher im allgemeinen sehr hohe Dosierungen mit entsprechenden toxischen Nebenwirkungen, siehe H. Breidthaupt, E. Küenzlen, (1983), «High dose methotrexate for osteosarcoma: toxicity and clinical results.» Oncology 40, 85–89.

Bekannt sind weiterhin die Bemühungen, cytostastisch wirksame Verbindungen chemisch so zu verändern, dass sie sich nach Verabreichung am Wirkort anreichern. Die Konzeption dieser Arbeitsrichtung geht von der bekannten Tatsache aus, dass ausgewählte Verbindungen bestimmter Strukturtypen zur Anreicherung in bestimmten Organen des lebenden Organismus tendieren, so dass durch eine Verknüpfung einer solchen organspezifischen Komponente mit einer cytostatisch aktiven Komponente deren Transport in das betroffene Organ gefördert werden könnte. Diese Bemühungen haben jedoch bisher – zumindest in der überwiegenden Anzahl der Fälle – nicht zum Erfolg geführt. Im allgemeinen sind die chemisch veränderten Substanzen nicht mehr organspezifisch. Das gilt beispielsweise für den Versuch, Steroidhormone (insbesondere Östradiol und Testosteron) mit alkylierenden Gruppen zu koppeln, um so Tumoren in Geweben treffen zu können, die diese Hormone normalerweise anreichern, vgl. hierzu beispielsweise Journal of Medicinal Chemistry, 1979, Vol. 22, No. 2, 200–202. Auch beim Versuch, Lebertumoren mit alkylierenden Derivaten von Sulfonamiden zu behandeln, deren Grundkörper sich in der Leber anreichert, ging die Organspezifität durch die Derivatisierung verloren. Lediglich in nicht mit Sicherheit voraussehbaren Sonderfällen gelingt eine mehr oder weniger wirkungsvolle Verwirklichung des angegebenen Arbeitskonzepts, vgl. beispielsweise Arch. Pharm. (Weinheim) 311, 184–195 (1978).

Die Verwendung von N-Lost-Derivaten ist für die Behandlung unterschiedlicher Neoplasmien bekannt und üblich. Chlorambucil – in systematischer Bezeichnung 4-(4-Bis-(2-chlorethyl)-amino)-phenylbuttersäure – ist ein seit Jahren therapeutisch genutzter Stoff, der allerdings gegenüber Knochentumoren unspezifisch ist. Eine ganze Reihe strukturanaloger Verbindungen sind zum gleichen Einsatzzweck vorgeschlagen worden.

Bekannt ist schliesslich auch, dass bestimmte polyfunktionale Diphosphonsäuren – insbesondere Alkandiphosphonsäuren mit einer 1-Hydroxy-1,1-diphosphonsäure-Gruppierung – die Eigenschaft besitzen, auf Apatit aufzuziehen und sich im Knochengerüst anzureichern. Genannt seien hier beispielsweise die AT-PS 307 135 sowie die DE-PS 2 405 254.

Aus der europäischen Patentanmeldung 84 822 sind bereits Diphosphonsäurederivate mit antiinflammatorischer und antiphlogistischer Wirksamkeit bekannt. Diese Anmeldung enthält jedoch keinen Hinweis auf die Möglichkeit durch Kombination von N-Lost-Derivaten und Phosphonsäuregruppen Cytostatica mit geringer Mutagenität und Organspezifität zum Knochen zu erhalten. Bekannt ist auch das Cytostaticum Chlorambucil (Journal of Medical Chemistry, 22, No. 10, October 1979, Seiten 1267–1269). Es wurde jedoch bisher nicht versucht, dieses Cytostaticum mit Phosphonsäuregruppen zu modifizieren.

Die Erfindung geht von der Aufgabe aus, eine Verbessung der chemotherapeutischen Möglichkeiten bei der Behandlung von Knochentumoren zu schaffen. Insbesondere wurde nach einem Cytostatikum gesucht, das eine nachweisliche Anreicherung der Substanz im Knochen bzw. Knochentumor mit der cytostatischen Wirksamkeit dieser Substanz verbindet.

Die Lehre der Erfindung geht von der Feststellung aus, dass durch die Verknüpfung der beiden zuvor geschilderten Wirkungsmechanismen in einer Verbindung – Apatit-Affinität durch Diphosphonsäuregruppierung und cytostatische Wirkung durch die alkylierende N-Lost-Gruppe – unter Beibehaltung beider Wirkungen am Tumor verbesserte Ergebnisse erreicht werden können. Für das Gebiet der Behandlung von Knochentumoren wird damit ein Cytostatikum zur Verfügung gestellt, das die bisher bekannten therapeutischen Möglichkeiten deutlich übertrifft.

Gegenstand der Erfindung sind dementsprechend in einer ersten Ausführungsform die neuen chemischen Verbindungen, die die geschilderten Wirkungsprinzipien der Organspezifität und der cytostatischen Aktivität in sich vereinigen. In einer weiteren Ausführungsform betrifft die Erfindung das Verfahren zur Herstellung dieser neuen Verbindungen. Schliesslich betrifft die Erfindung die Verwendung dieser Verbindungen zur Behandlung von Knochentumoren bzw. pharmazeutische Zubereitungen mit einem Gehalt der neuen Verbindungen für den angegebenen Einsatzzweck.

Die neuen cytostatisch aktiven und gleichzeitig organspezifischen Komponenten sind neue 1-Hydroxy-1,1-diphosphonsäureverbindungen der allgemeinen Formel I

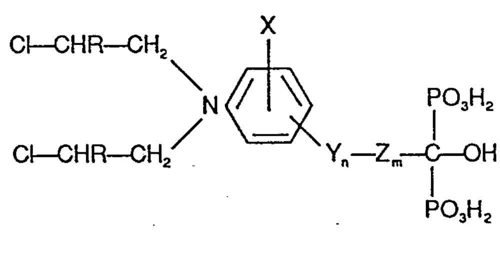
(I)

in der bedeuten

R = Wasserstoff und/oder Methyl

X = Wasserstoff, Halogen, Amino, das auch substituiert sein kann, Niedrigalkyl mit bis zu 5 C-Atomen, das auch mit Amino substituiert sein kann oder Niedrigalkoxy mit bis zu 5 C-Atomen.

Y = O, S oder NH,

Z = Niedrigalkylen, mit bis zu 5 C-Atomen
m und n = 0 oder 1 mit der Massgabe, dass für n = 1 auch m = 1 ist,
und ihre pharmakologisch verträglichen Salze.

Der über Y an den aromatischen Ring gebundene Molekülteil wird häufig in p-Stellung zur Lost-Gruppierung vorliegen, er kann aber auch als Substituent in m- bzw. in o-Stellung zugegen sein. In einer Gruppe bevorzugter Verbindungen bedeutet R in beiden Fällen Wasserstoff. Es kann weiterhin bevorzugt sein, dass auch der Rest X Wasserstoff bedeutet. Bevorzugte Halogensubstituenten für X sind Fluor, Chlor oder Brom. Bevorzugte niedere Alkylreste für X weisen nicht mehr als 5, insbesondere nicht mehr als 3 C-Atome auf. Charakteristische Beispiele sind der Methyl- bzw. der Ethylrest.

Der Substituent X kann Aminogruppen enthalten, insbesondere im Fall aminosubstituierter Alkylreste, oder auch selbst eine Aminogruppe darstellen.

In einer Gruppe bevorzugter Verbindungen der allgemeinen Formel I ist der Zahlenwert von n = 0, so dass das Bindeglied Y entfällt. Stattdessen greift der Alkylenrest Z unmittelbar am aromatischen Ring an. Dieser geradkettige oder verzweigte und gegebenenfalls substituierte Rest Z enthält nicht mehr als 5 C-Atome. Dabei ist es weiterhin bevorzugt, dass im geraden Kettenstück nicht mehr als 5 C-Atome und insbesondere nicht mehr als 3 C-Atome vorliegen. Als ein möglicher Substituent an Z ist wiederum die Aminogruppe in Betracht zu ziehen. Bevorzugte Reste für Z sind die folgenden:

$$-CH_2-,\ -(CH_2)_2-,\ -(CH_2)_3-,\ -CHCH_3-CH_2-,$$

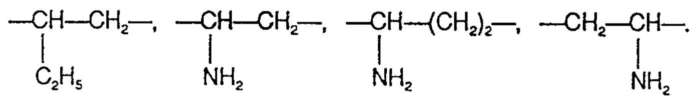

Wie angegeben können m und n die Zahlenwerte 0 oder 1 darstellen, jedoch mit der Massgabe, dass für n = 1 – d.h. für das tatsächliche Vorliegen eines der Reste O, S oder NH – dann auch m = 1 ist. Wie ebenfalls bereits angegeben, kann in einer bevorzugten Klasse von Verbindungen der Rest Y entfallen – d.h. n = 0 sein –, hier ist es dann auch möglich, dass m den Wert von 0 annimmt. In einer bevorzugten Klasse von erfindungsgemässen Verbindungen hat jedoch m die Bedeutung von 1.

Eine besonders wichtige neue Verbindung im Rahmen der allgemeinen Formel ist die 4-(4-(Bis-(2-chlorethyl)-amino)-phenyl)-1-hydroxybutan-1,1-diphosphonsäure, die im folgenden der Kürze halber als «BAD» bezeichnet wird.

In den Rahmen der Erfindung fallen dabei die freien Säuren der allgemeinen Formel I und insbesondere die «BAD». Für die pharmakologische Anwendung geeignet können aber insbesondere diese Säuren in Form ihrer pharmakologisch unbedenklichen Salze sein. Beispiele für solche Salze sind Alkali-, Erdalkali- und/oder Ammoniumsalze, wie Natrium, Kalium-, Magnesium-, Ammonium- und substituierte Ammoniumsalze. Sowohl partielle Salze, in denen nur ein Teil der aziden Protonen durch andere Kationen ersetzt ist, als auch Vollsalze fallen in den Rahmen der Erfindung. Bevorzugt können annähernd neutral reagierende Salze (pH etwa 5–9) sein. Mischungen verschiedener Salze als auch Mischungen der freien Säuren mit Salzen fallen in den Rahmen der Erfindung. Die freien Säuren selber bilden vermutlich mit ihrem tertiären N-Atom an der N-Lost-Gruppierung ein partielles inneres Salz.

Auch Salze aus den erfindungsgemässen Substanzen und Säuren können eingesetzt werden. Insbesondere können hierbei Salze physiologisch gut verträglicher Säuren eingesetzt werden. So sind Salze mit Halogenwasserstoffen, wie HCl, HBr oder HI geeignet. Weiterhin geeignet sind Salze mit Schwefelsäure oder Phosphorsäure.

Die neuen Verbindungen der allgemeinen Formel I und insbesondere das neue BAD bzw. seine Salze sind sowohl cytostatisch wirksam als auch zur Anreicherung in Knochentumoren befähigt. Die Substanz zeigt im sog. Ames-Test nur sehr geringes mutagenes Potential. Es ist bekannt, dass andere Alkylantien, die zur Chemotherapie des Krebses eingesetzt werden, starke Mutagene sind. Da Mutagene ihrerseits im Verdacht stehen, Krebs auszulösen, ist hier ein zusätzlicher beachtlicher Vorteil für die neue Verbindung zu sehen. Unabhängig von ihrer cytostatischen Wirksamkeit erweist sich die Substanz als schwach antimikrobiell wirksam, vorzugsweise gegen grampositive Keime, also Kokken, wie Staph. aureus und Strept. mutans, bei einer Hemmkonzentration von 1000 ppm.

BAD besitzt die Strukturformel

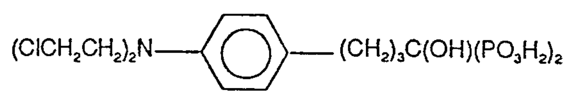

Zu seiner Herstellung geht man von Chlorambucil, d.h. von der 4-(4-Bis-(2-chlorethyl)-amino)-phenylbuttersäure aus. In an sich bekannter Reaktion wird die endständige Carboxylgruppe des Chlorambucils in den entsprechenden 1-Hydroxy-1,1-diphosphonsäurerest umgewandelt. Dieser Aspekt der Erfindung macht sich die bekannten Verfahren zur Herstellung von 1-Hydroxyal-

kan-1,1-diphosphonsäuren durch Reaktion der entsprechenden im Alkanrest substituierten oder unsubstituierten aliphatischen Carbonsäuren mit $H_3PO_3$ und einer Phosphor-Halogen-Verbindung unter Umwandlung der endständigen Carboxylgruppe zur 1-Hydroxy-1,1-diphosphonsäuregruppierung zunutze. Aus der umfangreichen einschlägigen Literatur sei beispielsweise verwiesen auf die DE-PSen 2 130 794, 2 658 961, 2 943 498 sowie auf die DE-OSen 2 702 631 und 3 151 038.

Bekannt ist danach, substituierte und/oder unsubstituierte Alkancarbonsäuren mit $H_3PO_3$ und Phosphor-Halogen-Verbindungen – insbesondere entsprechenden Chlorderivaten des Phosphors – unter Ausbildung der entsprechenden 1-Hydroxy-1,1-diphosphonsäurekomponenten umzuwandeln. Als Phosphor-Halogen-Verbindungen geeignet sind insbesondere $PCl_3$, $POCl_3$ und/oder $PCl_5$. Es kann dabei auch mit wasserfreier Phosphorsäure – sog. «kristallisierter Phosphorsäure» gearbeitet werden. vgl. hierzu DE-OS 3 151 038.

In einem komplexen Reaktionsmechanismus findet die Umwandlung der Carboxylgruppe zur Diphosphonsäuregruppierung statt. Die Phosphor-Halogen-Verbindung dient dabei in erster Linie als wasserabspaltendes Agens, das dabei anfallende Hydrolyseprodukt der phosphor-Halogen-Komponente kann dann aber auch seinerseits in die Substitutionsreaktion eingreifen. Werden ausschliesslich Phosphor-Halogen-Komponenten des 5wertigen Phosphors eingesetzt, dann wird die $H_3PO_3$ in einer bevorzugten Ausführungsform des erfindungsgemässen Verfahrens wenigstens in der stöchiometrisch benötigten Menge – 2 Mol $H_3PO_3$ pro Mol Chlorambucil – verwendet. Leitet sich die Phosphor-Halogen-Verbindung jedoch wenigstens anteilsweise von 3wertigem Phosphor ab – insbesondere im Falle des $PCl_3$ –, dann kann die $H_3PO_3$ in unterstöchiometrischen Mengen zum Einsatz kommen. Geeignet sind hier beispielsweise 1 bis 2 Mol $H_3PO_3$ pro Mol Chlorambucil. Die bei der Reaktion entstehenden Hydrolyseprodukte des $PCl_3$ liefern die zur Reaktion zusätzlich benötigten Mengen des Reaktanten mit 3wertigem Phosphor.

Das Herstellungsverfahren kann in an sich bekannter Weise in Gegenwart oder in Sonderfällen auch in Abwesenheit von inerten Verdünnungsmitteln erfolgen. Das Arbeiten in Gegenwart von inerten, bei Reaktionstemperatur flüssigen Verdünnungsmitteln ist bevorzugt.

In einer bevorzugten Ausführungsform wird das substituierte Buttersäure-Ausgangsmaterial von der Reaktion mit $H_3PO_3$ und dem wasserabspaltenden Agens in einem inerten Lösungsmittel, z.B. Halogenkohlenwasserstoffen, wie Chlorbenzol, gelöst bzw. suspendiert und mit einer Mineralsäure, insbesondere mit gasförmigem HCl versetzt. Zweckmässig wird wenigstens eine zur Salzbildung an der N-Lost-Gruppierung stöchiometrisch ausreichende Menge HCl verwendet, wobei es zweckmässig sein kann, in einem mit HCl-Gas gesättigten Reaktionsmedium zu arbeiten. Es hat sich herausgestellt, dass durch diese Massnahme eine Ausbeute-Steigerung an BAD möglich wird. In einer besonders geeigneten Ausführungsform des erfindungsgemässen Verfahrens wird Chlorambucil in einem inerten Lösungsmittel gelöst vorgelegt, die Lösung mit HCl-Gas gesättigt, dann $H_3PO_3$ im Mol-Verhältnis 1 bis 2 Mol $H_3PO_3$ je Mol Chlorambucil zugegeben und schliesslich – zweckmässigerweise unter Rühren – $PCl_3$ nach und nach zugegeben.

In der Stufe der Sättigung des Reaktionsansatzes mit gasförmigem HCl werden zweckmässigerweise Temperaturen bis höchstens 40 °C eingehalten. Die Umsetzung des Ausgangsmaterials mit phosphoriger Säure erfolgt dann bevorzugt im Temperaturbereich von etwa 60–110 °C, insbesondere im Bereich von etwa 70–100 °C.

Das primär anfallende Reaktionsprodukt aus der Umsetzung des Chlorambucils mit $H_3PO_3$ und der wasserabspaltenden Phosphor-Halogen-Verbindung wird in an sich bekannter Weise durch Versetzen mit Wasser hydrolysiert. Das anfallende rohe BAD-Produkt kann durch Versetzen mit wassermischbaren Lösungsmitteln, beispielsweise Aceton, gewünschtenfalls unter Zugabe von Wasser ausgefällt und gereinigt werden. Reines BAD fällt üblicherweise als weisses Pulver an, das gewünschtenfalls in an sich bekannter Weise in seine Salze überführt werden kann.

Die hier am speziellen Beispiel der Umsetzung von Chlorambucil gemachten Herstellungsangaben für die neuen Verbindungen der allgemeinen Formel I gelten ganz allgemein. Dementsprechend ist ein weiterer Gegenstand der Erfindung das Verfahren zur Herstellung dieser neuen 1-Hydroxy-1,1-diphosphonsäureverbindungen der allgemeinen Formel I und/oder ihren Salzen, wobei dieses Verfahren dadurch gekennzeichnet ist, dass man die Carboxylgruppe in 1-Stellung von substituierten Carbonsäuren der allgemeinen Formel II

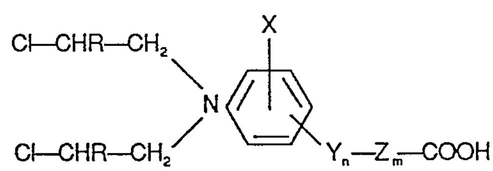

(II)

in der R, X, Y, Z, m und n die angegebene Bedeutung haben, in an sich bekannter Reaktion mit $H_3PO_3$ und einer wasserabspaltenden Verbindung, insbesondere einer Phosphorhalogenverbindung bzw. wasserfreier Phosphorsäure, zum 1-Hydroxy-1,1-disphosphonsäurerest umwandelt und die freie Säure gewünschtenfalls in ihre Salze überführt. Die am Beispiel der Umwandlung des Chlorambucils angegebenen bevorzugten Verfahrensmassnahmen gelten sinngemäss für die Herstellung der neuen Verbindungsklasse im Umfange der Lehre der allgemeinen Formel I.

Die erfindungsgemässen pharmakologischen Zubereitungen mit cytostatischer Wirksamkeit und insbesondere erhöhter Affinität zu Knochentumoren enthalten die Verbindungen der allgemeinen Formel I bzw. ihre Salze und insbesondere die BAD bzw. seine Salze in üblichen Darreichungsformen, wie sie zur oralen, subkutanen, intramuskulären oder intravenösen Behandlung geeignet sind. Die Substanzen können zur Verabreichung in Tabletten, Pillen, Kapseln oder Injektionslösungen formuliert werden. Neben der pharmakologisch wirksamen Substanz können übliche Hilfs- und/oder Trägerstoffe in flüssiger und/oder fester Form vorliegen. Der doppelte Wirkungsmechanismus der neuen Verbindung lässt sich einerseits durch szintigraphische Messungen zur Tumoranreicherung und andererseits durch Prüfung der cytostatischen Wirksamkeit, insbesondere an der Bestimmung des Tumorvolumens und/oder der Überlebenszeit von Versuchstieren belegen.

Beispiel 1

In einer Inertgasatmosphäre werden 0,033 Mol Chlorambucil unter Feuchtigkeitsausschluss in 75 ml Chlorbenzol gelöst und 30 Min. Chlorwasserstoff eingeleitet, wobei ein weisser Niederschlag ausfällt. Die Temperatur wird dabei unter 40°C gehalten. Anschliessend werden 0,05 Mol Phosphorige Säure zugegeben, auf 75°C erwärmt und langsam 0,05 Mol Phosphortrichlorid zugetropft. Nach beendetem Zutropfen werden 30 Min. bei 75–80°C und weitere vier Stunden bei 100°C nacherhitzt.

Das abgekühlte Reaktionsprodukt wird dann mit 50 ml Wasser hydrolysiert, wobei eine hellbraune Paste entsteht, von der die Chlorbenzolphase abgetrennt wird und die mit Aceton zu einem weissen Pulver verrieben wird. Ein farbloses kristallines Produkt wird abfiltriert und über Phosphorpentoxid getrocknet.

Ausbeute: 50%.

Analyse:

| | | | | |
|---|---|---|---|---|
| C 37.1 | H 5.40 | P 13.4 | N 2.83 | Cl 15.9 |
| (37.33) | (5.11) | (13.78) | (3.11) | (15.78) |

Beispiel 2

Die akute Toxizität des BAD (intravenöse Gabe als Dinatriumsalz) wird an der Maus (oral) mit $DL_{50}$ mg/kg > 625 und an der SD-Ratte (i.v.) mit folgenden Werten bestimmt:

| $DL_{10}$ mg/kg | $DL_{50}$ mg/kg | $DL_{50}$ mmol/kg |
|---|---|---|
| 82 | 146 | 0,32 |

**Patentansprüche**

1. Neue 1-Hydroxy-1,1-diphosphonsäureverbindungen der allgemeinen Formel I

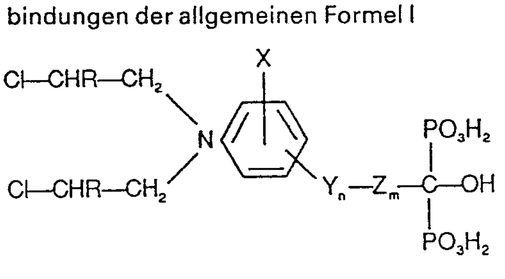

(I)

in der bedeuten

R = Wasserstoff und/oder Methyl

X = Wasserstoff, Halogen, Amino, das auch substituiert sein kann, Niedrigalkyl mit bis zu 5 C-Atomen, das auch mit Amino substituiert sein kann oder Niedrigalkoxy mit bis zu 5 C-Atomen.

Y = O, S oder NH,

Z = Niedrigalkylen, mit bis zu 5 C-Atomen

m und n = 0 oder 1 mit der Massgabe, dass für n = 1 auch m = 1 ist und ihre pharmakologisch verträglichen Salze.

2. 1-Hydroxy-1,1-diphosphonsäureverbindungen der allgemeinen Formel I bzw. ihre pharmakologisch verträglichen Salze, in der R, X, Z, m und n die folgende Bedeutung haben:

R und X = Wasserstoff, Z = geradkettiger Alkylenrest mit bis zu 5 C-Atomen, m = 1 und n = 0.

3. 4-(4-(Bis-(2-chlorethyl)-amino)-phenyl)-1-hydroxybutan-1,1-diphosphonsäure (BAD) und ihre pharmakologisch verträglichen Salze.

4. Verfahren zur Herstellung der neuen 1-Hydroxy-1,1-diphosphonsäureverbindungen der allgemeinen Formel I und/oder ihren Salzen, dadurch gekennzeichnet, dass man die Carboxylgruppe in 1-Stellung von substituierten Carbonsäuren der allgemeinen Formel II

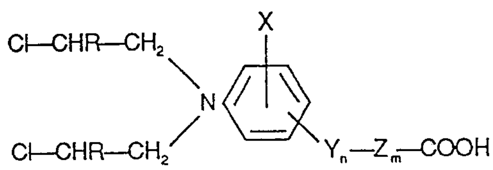

(II)

in der R, X, Y, Z, m und n die angegebene Bedeutung haben, in an sich bekannter Reaktion mit $H_3PO_3$ und einer wasserabspaltenden Verbindung, insbesondere einer Phosphorhalogenverbindung bzw. wasserfreier Phosphorsäure, zum 1-Hydroxy-1,1-diphosphonsäurerest umwandelt und die freie Säure gewünschtenfalls in ihre Salze überführt.

5. Verfahren nach Anspruch 4, dadurch gekennzeichnet, dass man zur Herstellung von BAD und/oder ihren Salzen die endständige Carboxylgruppe der 4-(4-Bis-(2-chlorethyl)-amino)phenylbuttersäure in an sich bekannter Reaktion mit $H_3PO_3$ und einer Phosphorhalogenverbindung, insbesondere $PCl_3$, $POCl_3$ und/oder $PCl_5$ bzw. wasserfreier Phosphorsäure, zum 1-Hydroxy-1,1-diphosphonsäurerest umwandelt und die freie Säure gewünschtenfalls in ihre Salze überführt.

6. Verfahren nach Ansprüchen 4 oder 5, dadurch gekennzeichnet, dass man der Reaktionszone vor der Umwandlung der Carboxylgruppe HCl zuführt, wobei vorzugsweise mindestens mit stöchiometrisch äquivalenten Mengen HCl – bezogen auf Carbonsäurederivat –, insbesondere aber in einem mit HCl gesättigten Reaktionsmedium gearbeitet wird.

7. Verfahren nach einem der Ansprüche 4 bis 6, dadurch gekennzeichnet, dass die Sättigung des Reaktionsmediums mit HCl bei Temperaturen nicht über 40°C vorgenommen wird, während zur Umwandlung der Carboxylgruppe bevorzugt

im Temperaturbereich von 60–110°C gearbeitet wird.

8. Pharmakologische Zubereitungen mit cytostatischer Wirksamkeit und insbesondere erhöhter Affinität zu Knochentumoren, dadurch gekennzeichnet, dass sie Verbindungen der allgemeinen Formel I und insbesondere 4-(4-(Bis-(2 chlorethyl)-amino)-phenyl-1-hydroxybutan-1,1-diphosphonsäure (BAD) und/oder ihre Salze enthalten.

## Claims

1. New 1-hydroxy-1,1-diphosphonic acid compounds corresponding to the following general formula

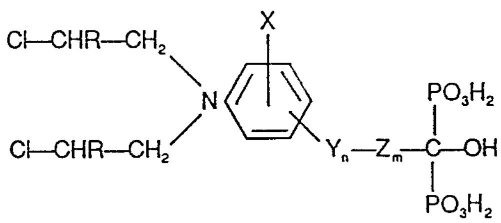
(I)

in which
R represents hydrogen and/or methyl,
X represents hydrogen, halogen, amino which may even be substituted, lower alkyl, which may even be substituted by amino, or lower alkoxy containing up to 5 carbon atoms,
Y = O, S or NH,
Z = lower alkylene containing up to 5 carbon atoms,
m and n = 0 or 1, with the proviso that, where n is 1, m is also 1, and pharmacologically compatible salts thereof.

2. 1-hydroxy-1,1-diphosphonic acid compounds corresponding to general formula I
in which R, X, Z, m and n have the following meanings:
R and X = hydrogen, Z = branched-chain alkylene radical containing up to 5 carbon atoms,
m = 1 and n = 0 and pharmacologically compatible salts thereof.

3. 4-(4-(bis-(2-chloroethyl)-amino)-phenyl) -1-hydroxy-butane-1,1-diphosphonic acid (BAD) and pharmacologically compatible salts thereof.

4. A process for producing the new 1-hydroxy-1,1-diphosphonic acid compounds corresponding to general formula I and/or salts thereof, characterized in that the carboxyl group in the 1-position of substituted carboxylic acids corresponding to the following general formula

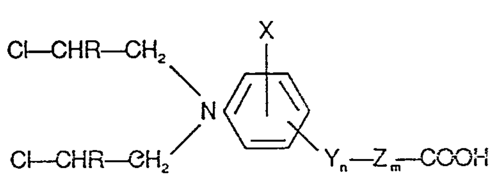
(II)

in which R, X, Y, Z, m and n are as defined above, is converted into the 1-hydroxy-1-diphosphonic acid residue in known manner by reaction with $H_3PO_3$ and a dehydrating compound, particularly a phosphorus-halogen compound or anhydrous phosphoric acid, and the free acid is optionally converted into its salts.

5. A process as claimed in Claim 4, characterized in that, to produce BAD and/or its salts, the terminal carboxyl group of 4-(4-bis-(2-chloroethyl)-amino)-phenyl butyric acid is converted into the 1-hydroxy-1,1-diphosphonic acid residue in known manner by reaction with $H_3PO_3$ and a phosphorus-halogen compound, particularly $PCl_3$, $POCl_3$ and/or $PCl_5$, or anhydrous phosphoric acid, and the free acid is optionally converted into its salts.

6. A process as claimed in Claim 4 or 5, characterized in that HCl is introduced into the reaction zone before conversion of the carboxyl group, at least stoichiometrically equivalent quantities, based on carboxylic acid derivative, of HCl preferably being used or, more preferably, the reaction being carried out in a reaction medium saturated with HCl.

7. A process as claimed in any of Claims 4 to 6, characterized in that saturation of the reaction medium with HCl is carried out at temperatures not exceeding 40°C, whereas conversion of the carboxyl group is preferably carried out at a temperature in the range from 60 to 110°C.

8. Pharmacological preparations showing cytostatic activity and, in particular, increased affinity for bone tumors, characterized in that they contain compounds corresponding to general formula I and, more particularly, 4-(4-bis-(2-chloroethyl)-aminophenyl-1-hydroxybutane-1,1-diphosphonic acid (BAD) and/or salts thereof.

## Revendications

1. Nouveaux composés d'acide 1-hydroxy-1,1-diphosphonique de formule générale I:

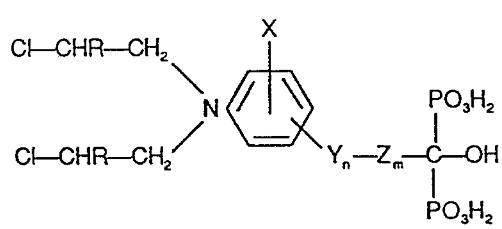
(I)

dans laquelle
R représente un atome d'hygrogène et/ou un groupe méthyle,
X représente un atome d'hydrogène, un atome d'halogène, un groupe amino qui peut également être substitué, un groupe alkyle inférieur contenant jusqu'à 5 atomes de carbone qui peut également être substitué par un groupe amino, ou un groupe alcoxy inférieur contenant jusqu'à 5 atomes de carbone,

Y représente O, S ou NH

Y représente un groupe alkylène inférieur contenant jusqu'à 5 atomes de carbone,

m et n représentent 0 ou 1, avec cette réserve que si n est égal à 1, m est également égal à 1, ainsi que leurs sels pharmacologiquement compatibles.

2. Composés d'acide 1-hydroxy-1,1-diphosphonique de formule générale I ou leurs sels pharmacologiquement compatibles, formule dans laquelle R, X, Z, m et n ont les significations suivantes:

R et X = atome d'hydrogène, Z = radical alkylène à chaîne droite contenant jusqu'à 5 atomes de carbone, m = 1 et n = 0.

3. Acide 4-(4-(bis-(2-chloréthyl)-amino)-phényl)-1-hydroxy-butane-1,1-diphosphonique (BAD) et ses sels pharmacologiquement compatibles.

4. Procédé de préparation des nouveaux composés d'acide 1-hydroxy-1,1-diphosphonique de formule générale I et/ou de leurs sels, caractérisé en ce qu'on transforme, en un radical d'acide 1-hyroxy-1,1-diphosphonique, le groupe carboxyle occupant la position 1 d'acides carboxyliques substitués de formule générale II:

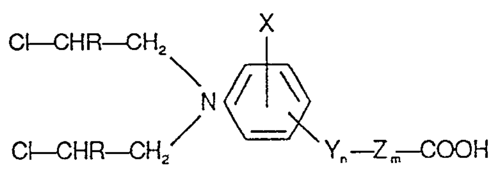

(II)

dans laquelle R, X, Y, Z, m et n ont les significations indiquées, dans une réaction connue en soi avec $H_3PO_3$ et un composé dissociant l'eau, en particulier, un composé halogéné de phosphore ou de l'acide phosphorique anhydre et, si on le désire, on transforme l'acide libre en ses sels.

5. Procédé selon la revendication 4, caractérisé en ce que, pour la préparation de BAD et/ou de ses sels, on transforme, en un radical d'acide 1-hydroxy-1,1-diphosphonique, le groupe carboxyle terminal de l'acide 4-(4-bis-(2-chloréthyl)-amino)-phényl-butyrique dans une réaction connue en soi avec $H_3PO_3$ et un composé halogéné de phosphore, en particulier, $PCl_3$, $POCl_3$ et/ou $PCl_5$ ou de l'acide phosphorique anhydre et, si on le désire, on transforme l'acide libre en ses sels.

6. Procédé selon la revendication 4 ou 5, caractérisé en ce que, avant la transformation du groupe carboxyle, on achemine du HCl à la zone réactionnelle, de préférence, en travaillant au moins avec des quantités stoechiométriquement équivalentes de HCl (rapporté au dérivé d'acide carboxylique) mais, en particulier, dans un milieu réactionnel saturé de HCl.

7. Procédé selon une des revendications 4 à 6, caractérisé en ce qu'on effectue la saturation du milieu réactionnel avec HCl à des températures ne dépassant pas 40 °C tandis que, pour la transformation du groupe carboxyle, on travaille, de préférence, à une température se situant dans l'intervalle allant de 60 à 110 °C.

8. Préparations pharmacologiques à activité cytostatique et ayant, en particulier, une plus haute affinité vis-à-vis de tumeurs osseuses, caractérisées en ce qu'elles contiennent des composés de formule générale I et, en particulier, l'acide 4-(4-(bis-(2-chloréthyl)-amino)-phényl)-1-hydroxy-butane-1,1-diphosphonique (BAD) et/ou ses sels.